Europäisches Patentamt

European Patent Office

Office européen des brevets

(19)

(11) Veröffentlichungsnummer : **0 465 968 B1**

(12) **EUROPÄISCHE PATENTSCHRIFT**

(45) Veröffentlichungstag der Patentschrift :
01.06.94 Patentblatt 94/22

(51) Int. Cl.$^5$ : **C02F 1/44,** // B01D61/02,
C07C255/00

(21) Anmeldenummer : **91110829.8**

(22) Anmeldetag : **29.06.91**

(54) **Verfahren zur Reinigung von Acrylnitril-Abwasser.**

(30) Priorität : **12.07.90 DE 4022222**

(43) Veröffentlichungstag der Anmeldung :
**15.01.92 Patentblatt 92/03**

(45) Bekanntmachung des Hinweises auf die
Patenterteilung :
**01.06.94 Patentblatt 94/22**

(84) Benannte Vertragsstaaten :
**DE ES GB IT NL**

(56) Entgegenhaltungen :
CHEMICAL ABSTRACTS, Band 82, Nr. 26, 30.
Juni 1975, Seite 264, ZusammenfassungNr.
174959c, Columbus, Ohio, US; & JP-A-74 97
457 (ASAHI CHEMICAL INDUSTRY CO., LD)
14-09-1974
CHEMICAL ABSTRACTS, Band 86, Nr. 16, 18.
April 1977, Seite 275, ZusammenfassungNr.
110830d, Columbus, Ohio, US; M.K. GUPTA:
"Development of a mobile treatmentsystem
for handling spilled hazardous materials"

(56) Entgegenhaltungen :
DESALINATION, Band 24, Nr. 1/2/3, Januar
1978, Seiten 155-173, ElsevierScientific Publishing Co., Amsterdam, NL; P. MAPPELLI et al.:
"Membraneprocesses used for the treatment
of industrial effluents"

(73) Patentinhaber : **EC ERDÖLCHEMIE GMBH**
**Alte Strasse 201**
**D-50769 Köln (DE)**

(72) Erfinder : **Herwig, Jens, Dr.**
**Nerzweg 2**
**W-5000 Köln 40 (DE)**
Erfinder : **Stüwe, Arnd, Dr.**
**Berta-von-Suttner-Strasse 34**
**W-5090 Leverkusen (DE)**
Erfinder : **Grub, Joachim, Dr.**
**Ostpreussenallee 10**
**W-4047 Dormagen (DE)**

(74) Vertreter : **Zobel, Manfred, Dr. et al**
**BAYER AG**
**Konzernverwaltung RP**
**Patente Konzern**
**D-51368 Leverkusen (DE)**

EP 0 465 968 B1

**Beschreibung**

Acrylnitril (ACN) ist ein industrielles Großprodukt, das fast ausschließlich durch Ammoxidation von Propen gemäß folgender Gleichung hergestellt wird:

$$CH_2=CH-CH_3 + NH_3 + 1\,1/2\,O_2 \rightarrow CH_2=CH-CN + 3\,H_2O\;.$$

Theoretisch entsteht so pro Tonne ACN ebenfalls etwa eine Tonne Wasser. Wegen der nicht 100 %igen Selektivität zum gewünschten ACN ist jedoch die Wassermenge größer und beträgt etwa 1,5 Tonnen oder mehr pro Tonne ACN. Dieses Abwasser ist mit unerwünschten Nebenprodukten belastet, zu denen Nikotinsäurenitril (Mol.-Gew. MW = 104), Fumarsäuredinitril (MW = 78), Bernsteinsäuredinitril (MW = 80), 3-Picolin (MW = 93) und 1-H-Pyrazol (MW = 68) gehören.

Zur Gewinnung des Acrylnitrils sind zwar beträchtliche interne Wasserkreisläufe erforderlich, beispielsweise zum Quenchen der heißen Reaktorabgase und bei der Abtrennung von Acrylnitril einerseits und Acetonitril und Blausäure andererseits durch Extraktivdestillation, wozu auch Abwässer eingesetzt werden können, die systemeigene Verunreinigungen aufweisen. In jedem Falle muß aber zur Vermeidung der Überfüllung dieser Wasserkreisläufe pro Zeiteinheit das gemäß obiger Formelgleichung entstehende Reaktionswasser aus dem gesamten Prozeß ausgeschleust werden. Es ist vielfach üblich, das letztendlich nach Durchlaufen der genannten internen Kreisläufe anfallende Abwasser einer Abwasserdestillation zu unterziehen, wobei ein Brüdenkondensat und ein stärker mit Abfallstoffen belasteter Sumpfablauf erhalten werden. An einer geeigneten Stelle des gesamten Verfahrens wird ein hoch mit Abfallstoffen belastetes Abwasser entnommen und der Abfallverbrennung zugeführt, um das Konzentrationsniveau an Abfallstoffen in den internen Kreisläufen auf einem geeigneten Niveau zu halten. Diese zur Abfallverbrennung gegebene Menge und das Brüdenkondensat müssen zusammen dem pro Zeiteinheit stets neu entstehenden Reaktionswasser entsprechen.

Das Brüdenkondensat enthält u.a. die oben namentlich aufgeführten Nebenprodukte. Eine weitere destillative Reinigung ist wegen der Wasserdampfflüchtigkeit dieser Nebenprodukte nicht möglich; das Brüdenkondensat muß also in dieser Form entsorgt werden. Hierzu gibt man dieses Brüdenkondensat beispielsweise in eine biologische Kläranlage. Die oben genannten organischen Stickstoffverbindungen als Nebenprodukte, zu denen noch Ammoniak und Cyanid kommen, stellen jedoch für eine Kläranlage auch bei adaptierten Mikroorganismen eine hohe Belastung dar und werden nicht vollständig abgebaut.

Gemäß heutigem Ökologieverständnis war es daher wünschenswert, den Anteil der genannten Nebenprodukte im Abwasser weiter abzusenken, um die nachfolgende biologische Kläranlage zu entlasten und effektiver arbeiten zu lassen. Es wurde überraschend gefunden, daß dieses Ziel dadurch erreichbar ist, daß Acrylnitril-Abwässer, die als Brüdenkondensat entnommen werden, einer Umkehrosmose an einer Membran unterzogen werden können.

Es ist zwar grundsätzlich bekannt, daß sich Abwässer durch Umkehrosmose reinigen lassen. Hierbei werden sowohl ionisch vorliegende Bestandteile als auch organische Neutralmoleküle zurückgehalten. Für ionische Bestandteile liegt die Rückhaltung im Abwasserkonzentrat im Bereich von 95 bis 99 %; die Rückhaltung ionischer Bestandteile spielt jedoch bei einem Brüdenkondensat nur eine untergeordnete Rolle. Für Neutralmoleküle ist diese Rückhaltung dagegen unter anderem stark vom Molekulargewicht abhängig. Als untere Grenze für eine effiziente Rückhaltung gilt im allgemeinen ein Molekulargewicht von wenigstens 90, besser wenigstens 100, während organische Neutralmoleküle mit niedrigerem Molekulargewicht durch die Membran bereits merklich durchschlagen.

Obwohl die Molekulargewichte der meisten der obengenannten Nebenprodukte unter 100 liegen, ist überraschend einer wirksame Zurückhaltung im Abwasserkonzentrat mit Hilfe der erfindungsgemäß angewendeten Umkehrosmose möglich. Diese Rückhaltung ist bereits bei einstufiger Anwendung der Umkehrosmose beträchtlich und steigt bei einer zweistufigen Durchführung auf 90 % und höher.

Es wurde nun ein Verfahren zur Reinigung des bei der Acrylnitril-Herstellung anfallenden Abwassers gefunden, das dadurch gekennzeichnet ist, daß man das Abwasser auf einen pH-Wert von 4 bis 9, bevorzugt von 5 bis 8, einstellt und es dann bei einer Temperatur von 10 bis 50°C, bevorzugt 20 bis 40°C, und einem Differenzdruck von 10 bis 80 bar, bevorzugt 15 bis 60 bar, einer Umkehrosmose an einer Membran unterzieht, wobei eine Strömungsgeschwindigkeit an der Membran von 0,5 bis 4 m/sec, bevorzugt 1 bis 3 m/sec, besonders bevorzugt 1,5 bis 2,5 m/sec, eingestellt wird.

Als erfindungsgemäß einsetzbare Membranen kommen alle zur Umkehrosmose geeigneten Membranen in Frage, ohne daß deren chemischer Aufbau eine signifikante Rolle spielt. In bevorzugter Weise eignen sich Thin-Film-Composite-Membranen. Solche Membranen können beispielsweise in Form von Flachmembranmodulen, Tubularmodulen oder Spiralwickelmodulen, bevorzugt in Form von Tubularmodulen oder Spiralwickelmodulen, eingesetzt werden. Beispiele sind etwa Wafilin WFC als Tubularmembran oder Wafilin WFT als Spiralwickelmodul.

Das erfindungsgemäße Verfahren kann grundsätzlich einstufig oder mehrstufig durchgeführt werden. Es ist selbstverständlich, daß bei mehrstufiger Fahrweise der Reinigungseffekt größer ist; jedoch steigt der Kostenaufwand bei mehrstufiger Fahrweise an. Wegen der meistens unzureichenden Reinigung bei nur einstufiger Fahrweise, ist die mehrstufe Fahrweise bevorzugt. Es wurde insbesondere gefunden, daß eine zweistufige Fahrweise ein günstiges Optimum zwischen Kosten und Erfolg darstellt.

Es hat sich weiter als vorteilhaft herausgestellt, bei einer mehrstufigen Fahrweise die in Fließrichtung des Abwassers weiter vorn liegenden Stufen, bevorzugt die erste Stufe, mit einem Tubularmodul auszurüsten und die weiter hinten liegenden Stufen, bevorzugt bei Zweistufigkeit die zweite Stufe, mit einem Spiralwickelmodul auszurüsten.

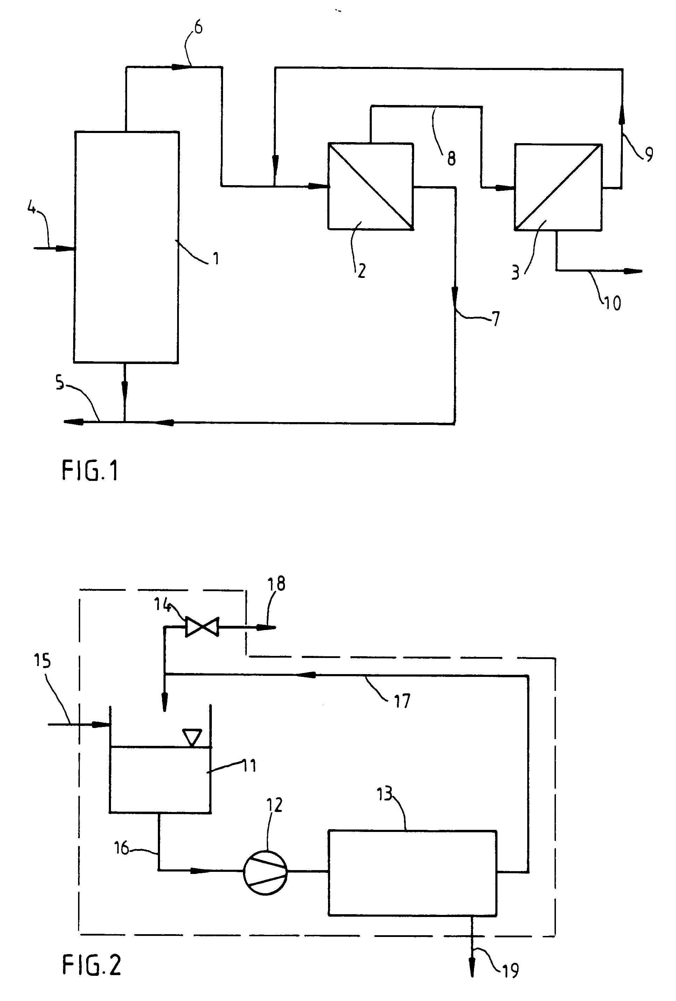

Anhand von Fig. 1 sei das erfindungsgemäße Verfahren in der bevorzugten zweistufigen Ausführung dargestellt: in Fig. 1 sind als Apparate dargestellt: eine Abwasserdestillationskolonne (1), eine erste Umkehrosmosestufe (2), bevorzugt ausgerüstet mit einem Tubularmodul, und eine zweite Umkehrosmosestufe (3), bevorzugt ausgerüstet mit einem Spiralwickelmodul. In Fig. 1 sind folgende Stoffströme dargestellt: ein Abwasserstrom (4) aus der ACN-Anlage, ein Sumpfablauf (5), der eine höhere Konzentration an Abfallstoffen aufzeigt als (4) und in die oben beschriebenen internen Wasserkreisläufe zurückkehrt, die Brüden (6), die kondensiert in (2) eingeführt werden; (2) entströmt ein erstes Konzentrat (7), welches dem Sumpfablauf (5) zugeschlagen wird, und ein erstes Permeat (8), welches in (3) eingespeist wird; (3) entströmt ein zweites Konzentrat (9), welches in die erste Umkehrosmosestufe (2) zurückgeführt wird, und ein zweites Permeat (10), das nunmehr mit erheblich verbessertem Erfolg in die biologische Abwasserklärung eingeleitet werden kann.

Es hat sich weiterhin als vorteilhaft erwiesen, bei mehrstufiger Umkehrosmose (bevorzugt bei zweistufiger Umkehrosmose) die verschiedenen Stufen (bevorzugt die beiden Stufen) unter etwas abweichenden, für jede Stufe optimierten Verfahrensbedingungen zu betreiben. Die wichtigste Optimierung betrifft den Konzentrierungsfaktor KF, der das Verhältnis der zulaufenden Abwassermenge zum entnommenen und im System noch befindlichen Konzentrat darstellt. Die Einstellung von KF sei anhand von Fig. 2 erläutert. Fig. 2 enthält als Apparate eine Abwasservorlage (11), ein Umwälzpumpe (12), ein Membranmodul (13) und ein Regelventil (14). Fig. 2 enthält als Stoffströme das zulaufende Abwasser (15), das von (11) über (12) nach (13) strömende Abwasser (16), den in der Umkehrosmosestufe intern umlaufende Konzentratstrom (17), das über (14) der Umkehrosmosestufe entnommene Konzentrat (18) und das der Umkehrosmosestufe entnommene Permeat (19). Die in Fig. 2 innerhalb der gestrichelten Linie befindlichen Apparate und Stoffströme entsprechen einer der Umkehrosmosestufen (2) bzw. (3) aus Fig. 1. Der Abwasserzulauf (15) in Fig. 2 entspricht dem Zulauf (6) bzw. (8) in Fig. 1. Das entnommene Konzentrat (18) in Fig. 2 entspricht (7) bzw. (9) in Fig. 1. Schließlich entspricht das entnommene Permeat (19) dem Permeat (8) bzw. (10) in Fig. 1.

KF ist unter stationären Bedingungen das Verhältnis der Menge (15) zur Menge von (18). Bei den in Fließrichtung weiter vorn liegenden Stufen, bevorzugt bei der ersten Stufe, wird ein KF von 2 bis 16, bevorzugt 6 bis 12, eingestellt. Bei den weiter hinten liegenden Stufen, bevorzugt bei Zweistufigkeit bei der zweiten Stufe, wird KF auf einen Wert von 2 bis 14, bevorzugt 3 bis 8, eingestellt.

Für das erfindungsgemäße Verfahren wird das zu reinigende Abwasser auf einen pH-Wert von 4 bis 9, bevorzugt von 5 bis 8, eingestellt. Diese pH-Wert-Einstellung geschieht mit Säuren oder Basen in einer grundsätzlich dem Fachmann bekannten weise. Beispielsweise werden hierzu $CO_2$, Essigsäure bzw. Natronlauge angewandt.

Die Temperatur für das erfindungsgemäße Verfahren liegt im Bereich von 10 bis 50°C, bevorzugt 20 bis 40°C, besonders bevorzugt 25 bis 35°C.

Die erfindungsgemäße Umkehrosmose wird mit einem Differenzdruck von 10 bis 60 bar zwischen der anzuströmenden Seite der Membran und der Permeatseite betrieben. In bevorzugter Weise wird ein Differenzdruck von 20 bis 50 bar angelegt. Es kann weiterhin günstig sein, bei einer mehrstufigen Durchführung des erfindungsgemäßen Verfahrens die zweite oder weitere Stufen bei einem tendenziell niedrigeren Druck zu fahren als die erste bzw. die weiter vorn liegenden Stufen. Hierzu sei ein Differenzdruck von 10 bis 50 bar, bevorzugt 25 bis 35 bar, genannt.

Der in der ersten Stufe oder in den ersten Stufen bei zweistufiger oder mehrstufiger Fahrweise tendenziell höhere Wert für KF ergibt ein geringeres, jedoch höher konzentriertes Konzentrat, wie es in die internen Wasserkreisläufe zurückgefahren oder der Abfallverbrennung zugeführt werden kann, und ein weniger gut gereinigtes Permeat. In der letzten oder in den letzten Stufen bei mehrstufiger Fahrweise wird KF tendenziell auf einen kleineren Wert eingestellt, wobei ein stärker gereinigtes Permeat erreicht wird und eine größere Menge Konzentrat, verglichen mit dem bei hohen Werten für KF, in die erste oder eine der vorangehenden Stufen zurückgeführt wird. Es kann daher sinnvoll sein, die Membranfläche von Stufe zu Stufe zu erhöhen, beispielsweise um jeweils 5 bis 10 % der Fläche der vorangegangenen Stufe.

Beispiele

Allgemeine Beschreibung

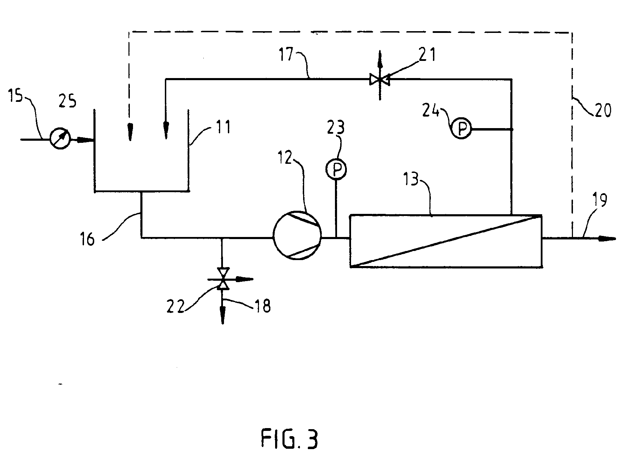

Die Beispiele wurden in der Apparatur nach Fig. 3 durchgeführt. Hierin bedeuten, in teilweiser Übereinstimmung mit Fig. 2, (11) eine Abwasservorlage, (12) eine Umwälzpumpe, (13) ein Membranmodul, (21) und (22) zwei Regelventile, (23) und (24) zwei Druckmeßstellen und (25) eine Wasseruhr. Die Stoffströme sind das zulaufende Abwasser (15), das dem Modul (13) zugeführte Abwasser (16) einschließlich der internen Stoffumläufe, der intern umlaufende Konzentratstrom (17), das entnommene Konzentrat (18), das entnommene Permeat (19) und der bis zur Einstellung eines stabilen Verhältnisses an der Membran ebenfalls umlaufende Permeatstrom (20). Das Gesamtkonzentratvolumen einschließlich der Vorlage (11) betrug 230 l, das Permeatvolumen 70 l.

Die Batch-Versuche (begrenzte Abwassermenge) der Beispiele 1-5 wurden wie folgt durchgeführt:

1. Befüllen der Apparatur mit Abwasser über die Wasseruhr (ca. 230 l);

2. Start der Umkehrosmose unter den jeweils angegebenen Bedingungen, wobei in der ersten Stunde auch das Permeat in die Vorlage (11) zurückgeführt wurde, um an der Membran stabile Verhältnisse einzustellen (KF = 1,3);

3. danach Ableiten des Permeats und weiterer Zulauf von Abwasser, bis über die Wasseruhr insgesamt 760 l Abwasser zugelaufen sind (steigende KF-Werte), dann Absperrung der Wasseruhr;

4. nach Absperrung des Abwassers wurden noch 154 l Permeat entnommen (KF = 10).

Beim Batch-Versuch in Beispiel 6 wurden 255 l eines Permeats aus Beispiel 7 als Abwasser eingesetzt und bis auf 44 l Konzentrat bei 211 l Permeatabnahme (KF = 5,8) eingedickt.

Beim kontinuierlichen Versuchslauf nach Beispiel 7 (unbegrenzte Abwassermenge) wurde zunächst bis zu KF = 10 aufkonzentriert, d.h. bis zum Durchsatz von 2300 l Abwasser und Erzeugung von 2070 l Permeat wurde kein Konzentrat abgenommen. Danach wurden je 100 l zugeführtem Abwasser 10 l Konzentrat abgenommen.

Beispiel 1 (Batch-Versuch)

Einsatz: ACN-Abwasser, mit Essigsäure auf pH 7 eingestellt
WFC-Membran (4 m$^2$), v = 2,5 m/s, $\Delta$P = 40 bar, T = 25°C

$$\text{MRHV} = \underline{M}\text{embran}\underline{r}\text{ück}\underline{h}\text{alte}\underline{v}\text{ermögen} = \frac{C_{\text{Konzentrat}} - C_{\text{Permeat}}}{C_{\text{Konzentrat}}}$$

TOC = $\underline{T}$otal $\underline{O}$rganic $\underline{C}$arbon
CSB = $\underline{C}$hemischer $\underline{S}$auerstof$\underline{f}$bedarf

| KF | | TOC mg/l | MRHV % | CSB mg/l | MRHV % | $NH_4^{\oplus}$ mg/l | MRHV % | Gesamtstickstoff mg/l | MRHV % | Flux l/m²h |
|---|---|---|---|---|---|---|---|---|---|---|
| 1 | Einsatz | 2890 | – | 2975 | – | 320 | – | 1115 | – | |
| 1,3 | Permeat | 530 | 82,7 | 521 | 86,3 | 10 | 96,8 | 227,4 | 84,6 | 43 |
| | Konzentrat | 3060 | | 3790 | | 380 | | 1480 | | |
| 2,0 | Permeat | 710 | 84,0 | 700 | 85,5 | 45 | 91,5 | 359,5 | 83,0 | 45 |
| | Konzentrat | 4450 | | 4835 | | 530 | | 2090 | | |
| 3,3 | Permeat | 870 | 86,7 | 930 | 88,9 | 69 | 93,3 | 446,5 | 83,9 | 35 |
| | Konzentrat | 6530 | | 8400 | | 970 | | 2770 | | |
| 5,0 | Permeat | 1000 | 88,4 | 1040 | 88,4 | 17 | 98,7 | 464 | 83,3 | 33 |
| | Konzentrat | 8640 | | 9000 | | 1260 | | 2780 | | |
| 10,0 | Permeat | 1550 | 87,9 | 2325 | 86,0 | 32 | 98,8 | 706 | 76,9 | 30 |
| | Konzentrat | 12800 | | 16600 | | 2650 | | 3062 | | |

Beispiel 2 (Batch-Versuch)

Einsatz: ACN-Abwasser, mit Essigsäure auf pH 7 eingestellt
WFC-Membran (4 m²), v = 2,5 m/s, $\Delta P$ = 40 bar, T = 35°C

| KF | | TOC mg/l | MRHV % | CSB mg/l | MRHV % | NH$_4^{\oplus}$ mg/l | MRHV % | Gesamtstickstoff mg/l | MRHV % | Flux l/m²h |
|---|---|---|---|---|---|---|---|---|---|---|
| 1 | Einsatz | 2760 | - | 3270 | - | 325 | - | 1070 | - | - |
| 1,3 | Permeat | 510 | 84,4 | 620 | 84,8 | 11 | 96,5 | 225,5 | 84,1 | 52 |
| | Konzentrat | 3270 | | 4070 | | 405 | | 1420 | | |
| 2,0 | Permeat | 740 | 84,4 | 740 | 87,5 | 21 | 96,9 | 322,5 | 84,0 | 47 |
| | Konzentrat | 4750 | | 5940 | | 640 | | 2020 | | |
| 3,3 | Permeat | 1180 | 84,3 | 1130 | 87,7 | 33 | 96,7 | 655 | 79,7 | 43 |
| | Konzentrat | 7060 | | 9205 | | 990 | | 3220 | | |
| 5,0 | Permeat | 1330 | 85,9 | 1175 | 90,3 | 41 | 97,0 | 536 | 84,2 | 41 |
| | Konzentrat | 9420 | | 12150 | | 1370 | | 3385 | | |
| 10,0 | Permeat | 1860 | 87,3 | 1790 | 89,3 | 34 | 98,5 | 1130 | 81,7 | 36 |
| | Konzentrat | 14690 | | 16600 | | 2230 | | 6190 | | |

Beispiel 3: (Batch-Versuch)

Einsatz: ACN-Abwasser, mit Essigsäure auf pH 7 eingestellt,
WFC-Membran (4 m²), v = 2,5 m/s, ΔP = 35 bar, T = 35°C

| KF | | TOC mg/l | MRHV % | CSB mg/l | MRHV % | NH$_4^+$ mg/l | MRHV % | Gesamtstickstoff mg/l | MRHV % | Flux 1/m²h |
|---|---|---|---|---|---|---|---|---|---|---|
| 1 | Einsatz | 2570 | - | 3620 | - | 275 | - | 1230 | - | - |
| 1,3 | Permeat | 630 | 80,0 | 653 | 82,4 | 50 | 84,3 | 296,5 | 74,7 | 47 |
| | Konzentrat | 3150 | | 3710 | | 318 | | 1170 | | |
| 2,0 | Permeat | 990 | 78,1 | 1050 | 82,2 | 22 | 95,7 | 450,5 | 73,6 | 53 |
| | Konzentrat | 4530 | | 5890 | | 510 | | 1705 | | |
| 3,4 | Permeat | 1300 | 74,9 | 1310 | 84,5 | 27 | 96,6 | 465,5 | 82,7 | 46 |
| | Konzentrat | 5630 | | 8470 | | 790 | | 2695 | | |
| 5,0 | Permeat | 1490 | 80,0 | 1510 | 86,3 | 40 | 96,4 | 778 | 74,4 | 42 |
| | Konzentrat | 7520 | | 11050 | | 1100 | | 3610 | | |
| 10,0 | Permeat | 440 | 96,1 | 1840 | 92,3 | 12 | 99,4 | 777,5 | 86,5 | 33 |
| | Konzentrat | 11400 | | 24000 | | 2030 | | 5760 | | |

Beispiel 4: (Batch-Versuch)

Einsatz: ACN-Abwasser, mit Essigsäure auf pH 7 eingestellt
WFC-Membran (4 m²), v = 2 m/s, $\Delta P$ = 40 bar, T = 35°C

7

| KF | | TOC mg/l | MRHV % | CSB mg/l | MRHV % | $NH_4^{\oplus}$ mg/l | MRHV % | Gesamtstickstoff mg/l | MRHV % | Flux $1/m^2 h$ |
|---|---|---|---|---|---|---|---|---|---|---|
| 1 | Einsatz | 2300 | – | 3635 | – | 295 | – | 1025 | – | – |
| 1,3 | Permeat | 520 | 80,7 | 625 | 83,9 | 5 | 98,2 | 96 | 92,1 | 51 |
| | Konzentrat | 2700 | | 3890 | | 339 | | 1210 | | |
| 2,0 | Permeat | 650 | 81,4 | 830 | 85,6 | 10 | 97,6 | 148 | 91,6 | 55 |
| | Konzentrat | 3500 | | 5760 | | 420 | | 1760 | | |
| 3,3 | Permeat | 1000 | 80,6 | 1320 | 84,3 | 14 | 97,8 | 305 | 88,2 | 50 |
| | Konzentrat | 5150 | | 8390 | | 650 | | 2575 | | |
| 5,0 | Permeat | 1100 | 83,6 | 1260 | 88,7 | 22 | 97,4 | 348,5 | 88,3 | 43 |
| | Konzentrat | 6700 | | 11150 | | 850 | | 2915 | | |
| 10,0 | Permeat | 1770 | 84,0 | 1795 | 90,2 | 33 | 98,2 | 608 | 84,0 | 38 |
| | Konzentrat | 10650 | | 18300 | | 1800 | | 3805 | | |

Beispiel 5: (Batch-Versuch)

Einsatz: ACN-Abwasser, mit Essigsäure auf pH 7 eingestellt,
WFC-Membran (4 m²), v = 3 m/s, ∆P = 40 bar, T = 35°C

8

Beispiel 6: (Batch-Versuch)

Einsatz: Permeat aus Beispiel 7
WFT-Membran, T = 25°C, ΔP = 30 bar,

| KF | | TOC mg/l | MRHV % | CSB mg/l | MRHV % | $NH_4^{\oplus}$ mg/l | MRHV % | Gesamtstickstoff mg/l | MRHV % | Flux l/m²h |
|---|---|---|---|---|---|---|---|---|---|---|
| 1 | Einsatz | 2300 | - | 3635 | - | 295 | - | 1025 | - | - |
| 1,3 | Permeat | 510 | 81,4 | 618 | 86,3 | 10 | 97,0 | 173,5 | 86,4 | 58 |
| | Konzentrat | 2740 | | 4495 | | 330 | | 1275 | | |
| 2,0 | Permeat | 760 | 81,2 | 930 | 86,3 | * | * | 545 | 70,6 | 60 |
| | Konzentrat | 4040 | | 6805 | | | | 1855 | | |
| 3,4 | Permeat | 1110 | 79,4 | 1255 | 87,0 | 27 | 96,9 | 484,5 | 82,7 | 54 |
| | Konzentrat | 5380 | | 10550 | | 870 | | 2795 | | |
| 5,0 | Permeat | 1270 | 81,3 | 1370 | 88,1 | 25 | 97,7 | 554,5 | 84,2 | 51 |
| | Konzentrat | 6800 | | 11550 | | 1100 | | 3500 | | |
| 10,0 | Permeat | 1920 | 82,2 | 1930 | 91,0 | 46 | 97,5 | 835 | 85,0 | 42 |
| | Konzentrat | 10800 | | 21400 | | 1810 | | 5575 | | |

* wurde nicht bestimmt

| KF | | TOC mg/l | MRHV % | CSB mg/l | MRHV % | $NH_4^{\oplus}$ mg/l | MRHV % | Gesamtstickstoff mg/l | MRHV % | Flux $l/m^2h$ |
|---|---|---|---|---|---|---|---|---|---|---|
| 1 | Einsatz | 1480 | - | 1765 | - | 41 | - | 590 | - | - |
| 2,0 | Permeat | 280 | 88,7 | 520 | 82,2 | 7 | 95,0 | 105 | 90,2 | 17,5 |
|  | Konzentrat | 2480 | | 2920 | | 95 | | 1075 | | |
| 4,0 | Permeat | 570 | 88,3 | 760 | 88,0 | 8 | 95,3 | 168 | 92,1 | 13,6 |
|  | Konzentrat | 4880 | | 6355 | | 170 | | 2125 | | |
| 5,8 | Permeat | 680 | 90,4 | 998 | 88,9 | 10 | 94,7 | 248 | 91,3 | 11,8 |
|  | Konzentrat | 7080 | | 8975 | | 190 | | 2840 | | |

Beispiel 7: (Vollkonti-Versuch, kontinuierliche Konzentratabnahme bei KF = 10)

Einsatz: ACN-Abwasser, mit Essigsäure auf pH 7 eingestellt; WFC-Membran (4 m²), T = 35°C, $\Delta P$ = 40 bar, v = 2,5 m/s; Laufzeit t in Tagen (d = days).

10

| t(d) | KF | | TOC mg/l | MRHV % | CSB mg/l | MRHV % | NH$_4^{\oplus}$ mg/l | MRHV % | Gesamtstickstoff mg/l | MRHV % | Flux l/m²h |
|---|---|---|---|---|---|---|---|---|---|---|---|
| 1 | 10 | Permeat | 1740 | 84,2 | 2260 | 84,4 | 37 | | 720 | 85,8 | 25 |
| | | Konzentrat | 11000 | | 14450 | | 1550 | 97,6 | 5070 | | |
| 2 | 10 | Permeat | 1420 | 88,1 | 1900 | 90,3 | 41 | | 602 | 89,1 | 23 |
| | | Konzentrat | 11900 | | 19600 | | 1850 | 97,8 | 5525 | | |
| 3 | 10 | Permeat | 1610 | 87,1 | 2040 | 90,8 | 49 | | 657 | 88,5 | 20 |
| | | Konzentrat | 12500 | | 22150 | | 1900 | 97,4 | 5700 | | |
| 4 | 10 | Permeat | 1510 | 89,0 | 1860 | 90,0 | 47 | | 608 | 89,4 | 17 |
| | | Konzentrat | 13680 | | 18650 | | 2168 | 97,8 | 5725 | | |
| 5 | 10 | Permeat | 1470 | 88,9 | 1530 | 91,1 | 50 | | 612 | 89,6 | 13 |
| | | Konzentrat | 13200 | | 18350 | | 2165 | 97,7 | 5890 | | |
| 6 | 10 | Permeat | 1640 | 86,6 | 1985 | 90,3 | 65 | | 630 | 89,6 | 10 |
| | | Konzentrat | 12200 | | 20500 | | 1700 | 96,2 | 6050 | | |
| 7 | 10 | Permeat | 1620 | 86,4 | 2050 | 90,3 | 62 | | 655 | 89,2 | 7 |
| | | Konzentrat | 11950 | | 21050 | | 1700 | 96,4 | 6060 | | |

**Patentansprüche**

1. Verfahren zur Reinigung des bei der Acrylnitril-Herstellung anfallenden Abwassers, dadurch gekennzeichnet, daß man das Abwasser auf einen pH-Wert von 4 bis 9, bevorzugt von 5 bis 8, einstellt und es dann bei einer Temperatur von 10 bis 50°C, bevorzugt 20 bis 40°C, und einen Differenzdruck von 10 bis 80 bar, bevorzugt 15 bis 60 bar, einer Umkehrosmose an einer Membran unterzieht, wobei eine Strömungsgeschwindigkeit an der Membran von 0,5 bis 4 m/sec, bevorzugt 1 bis 3 m/sec, besonders bevorzugt 1,5 bis 2,5 m/sec, eingestellt wird.

2. Verfahren nach Anspruch 1, dadurch gekennzeichnet, daß die Membran in Form eines Flachmembranmoduls, Tubularmoduls oder Spiralwickelmoduls, bevorzugt in Form eines Tubularmoduls oder Spiralwickelmoduls, eingesetzt wird.

3. Verfahren nach Anspruch 1, dadurch gekennzeichnet, daß die Umkehrosmose mehrstufig, bevorzugt zweistufig, durchgeführt wird.

4. Verfahren nach Anspruch 3, dadurch gekennzeichnet, daß die in Fließrichtung weiter vorn liegenden Stufen, bevorzugt die erste Stufe, mit einem Tubularmodul ausgerüstet werden und die weiter hinten liegenden Stufen, bevorzugt bei Zweistufigkeit die zweite Stufe, mit einem Spiralwickelmodul ausgerüstet werden.

5. Verfahren nach Anspruch 3, dadurch gekennzeichnet, daß der Konzentrierungsfaktor KF als Verhältnis der zulaufenden Abwassermenge zum entnommenen und noch im System befindlichen Konzentrat bei den in Fließrichtung weiter vorn liegenden Stufen, bevorzugt bei der ersten Stufe, auf KF = 2 bis 16, bevorzugt 6 bis 12, eingestellt wird und bei den weiter hinter liegenden Stufen, bevorzugt bei Zweistufigkeit bei der zweiten Stufe, auf KF = 2 bis 14, bevorzugt 3 bis 8, eingestellt wird.

**Claims**

1. Process for the purification of waste water resulting on the production of acrylonitrile, characterized in that the waste water is adjusted to a pH of 4 to 9, preferably of 5 to 8, and then, at a temperature of 10 to 50°C, preferably 20 to 40°C, and a differential pressure of 10 to 80 bar, preferably 15 to 60 bar, undergoes a reverse osmosis at a membrane, a flow velocity of 0.5 to 4 m/sec, preferably 1 to 3 m/sec, particularly preferably 1.5 to 2.5 m/sec, being established at the membrane.

2. Process according to Claim 1, characterized in that the membrane is used in the form of a flat membrane module, tubular module or spiral-wound module, preferably in the form of a tubular module or spiral-wound module.

3. Process according to Claim 1, characterized in that the reverse osmosis is carried out in multiple stages, preferably in two stages.

4. Process according to Claim 3, characterized in that the stages lying further upstream in the direction of flow, preferably the first stage, are equipped with a tubular module and the stages lying further downstream, preferably, in the case of two stages, the second stage, are equipped with a spiral-wound module.

5. Process according to Claim 3, characterized in that the concentration factor KF as a ratio of the amount of feed waste water to the concentrate taken off and the concentrate still situated in the system is established in the stages lying further upstream in the direction of flow, preferably in the first stage, at KF = 2 to 16, preferably 6 to 12 and in the stages lying further downstream, preferably, in the case of two stages, in the second stage, at KF = 2 to 14, preferably 3 to 8.

**Revendications**

1. Procédé d'épuration des eaux usées obtenues dans la production de l'acrylonitrile, caractérisé en ce qu'on ajuste le pH des eaux usées à une valeur de 4 à 9, de préférence de 5 à 8, et on soumet ensuite les eaux usées, à une température de 10 à 50°C, de préférence de 20 à 40°C et à une pression différentielle de 10 à 80 bars, de préférence de 15 à 60 bars, à une osmose inverse sur une membrane, en réglant alors une vitesse d'écoulement au niveau de la membrane de 0,5 à 4 m/s, de préférence de 1 à 3 m/s, notamment de 1,5 à 2,5 m/s.

2. Procédé suivant la revendication 1, caractérisé en ce que la membrane est utilisée sous forme d'un module à membrane plane, d'un module tubulaire ou d'un module à enroulement spiral, de préférence sous forme d'un module tubulaire ou d'un module à enroulement spiral.

3. Procédé suivant la revendication 1, caractérisé en ce que l'osmose inverse est conduite en plusieurs étapes, de préférence en deux étapes.

4. Procédé suivant la revendication 3, caractérisé en ce que les étages situés plus en avant dans la direction d'écoulement, de préférence le premier étage, sont équipés d'un module tubulaire et les étages situés plus en arrière, de préférence le second étage lorsqu'il y a deux étages, sont équipés d'un module à enroulement spiral.

5. Procédé suivant la revendication 3, caractérisé en ce que le facteur de concentration FC en tant que rapport de la quantité amenée d'eaux usées à la phase concentrée prélevée et se trouvant encore dans le système est ajusté à un FC de 2 à 16, de préférence de 6 à 12 dans les étages situés plus en avant dans la direction d'écoulement, de préférence dans le premier étage et à un FC de 2 à 14, de préférence de 3 à 8 dans les étages se trouvant plus en arrière, de préférence dans le second étage lorsqu'il y a deux étages.

FIG.1

FIG.2

FIG. 3